Europäisches Patentamt

European Patent Office

Office européen des brevets

(19)

(11) **EP 1 174 104 A1**

(12) # EUROPEAN PATENT APPLICATION

(43) Date of publication:
**23.01.2002 Bulletin 2002/04**

(51) Int Cl.7: **A61F 15/00**

(21) Application number: **00115724.7**

(22) Date of filing: **21.07.2000**

(84) Designated Contracting States:
**AT BE CH CY DE DK ES FI FR GB GR IE IT LI LU MC NL PT SE**
Designated Extension States:
**AL LT LV MK RO SI**

(71) Applicant: **The Procter & Gamble Company**
**Cincinnati, Ohio 45202 (US)**

(72) Inventors:
• **Costea, Helene Karin**
**67550 Worms (DE)**

• **Gagliardi, Ivano**
**65123 Pescara (IT)**
• **Long, Robert Edward**
**61476 Kronberg i.Ts. (DE)**
• **Pretz, Kathrin**
**65200 Wiesbaden (DE)**

(74) Representative: **Kremer, Véronique et al**
**Procter & Gamble Service GmbH Sulzbacher Strasse 40**
**65824 Schwalbach am Taunus (DE)**

(54) **Package for absorbent articles**

(57) The present invention provides a package containing absorbent articles having different colors. The package includes at least one first absorbent article packaged within the package and having a first color and at least one second absorbent article packaged within the package and having a second color, the second color being visually distinct from the first color. Preferably the package contains feminine protection absorbent articles, preferably pantiliners and/or sanitary napkins, being preferably either black, transparent, and/ or white.

Fig. 1

Printed by Jouve, 75001 PARIS (FR)

EP 1 174 104 A1

**Description**

Field of the Invention

The present invention relates to a package containing absorbent articles having different colors.

Such package has the advantage of providing absorbent articles, preferably pantiliners and/or sanitary napkins, of at least two different colors to give more freedom to the wearers as to the choice of undergarment when discretion is desired. Providing absorbent articles with different colors has the benefit of not becoming easily apparent by visual inspection when an undergarment of corresponding color is worn.

Background of the Invention

It has historically been common that absorbent articles, in general including diapers, adult incontinence products, underarm sweat products, collar inserts, sanitary napkins and pantiliners are provided in a color communicating a hygienic condition. This conventionally resulted in white or predominantly white articles. This did fit well with the historically predominant undergarment color in which these articles are worn namely white undergarments. As a result white pantiliners or white sanitary napkins are not easily recognizable when used in such white undergarments.

Very recently fashion has led women more frequently to use other colors than white undergarments. This has been dictated not only by fashion itself but also as a result of the development of clothing which has a certain translucency and allows the color of the undergarment to be recognized. Furthermore very recently so-called G-String undergarments or "Tanga-Slips" have become widely used in particular by a younger generation women. These so-called string tangas are particularly used to prevent easy recognition of the contour of the undergarment through tight fitting clothes. There is thus a need for absorbent articles like sanitary napkins or pantiliners which match the color of not only the undergarments but also the garments in order to prevent a color-based recognition of the presence of such articles (under the assumption that modern clothing can be translucent). For sanitary napkins or pantiliners having so-called wings which are folded around the outside of the undergarment this is even more important since the wing part of the sanitary napkin or pantiliner is on purpose folded onto the outside of the undergarment and therefore easily visible, depending on the clothing worn over the undergarment.

Also woman nature is such that women love changes not only in their clothing but also undergarments which often match the clothing color, especially in the instance where the clothing is translucent. Thus there is a need of providing absorbent articles of different colors matching the color of the undergarment on a daily basis, for example one color one day and another color the subsequent day.

Conventionally absorbent articles are sold in packages containing ten to forty or even more, identical absorbent articles like sanitary napkins or pantiliners having a given geometry and absorbency (cf. currently white available absorbent articles on the market). In order to provide absorbent articles of a variety of colors, multiplied by the number of different product sizes to cater for the different needs of the user would mean for a manufacturer to have an extremely large number of different packages which have to be stocked. At the same time the shops in which such products would be sold would have to provide enormous space in order to stock and offer each variety for the potential customers. And more importantly the consumer would be somehow obliged to purchase a package containing for example only pantiliners of one color, this may be considerably more than a consumer is willing to spend if she either only needs a small quantity of such a colored product, or if she wishes to experiment by trying such a product that she has never used before. Also as for the shop space, such packages might be fairly bulky and take up a relatively large space in consumer's shelves or closets, should the consumer want to wear every day undergarment of different colors, or even undergarments of different colors within a single day/night.

Thus as the number of colors is inevitably very large, since in particular black, skin color, dark blue, light blue, red, green, yellow, to just cite some of the colors of the undergarments, are broadly available, it is an object of the present invention of providing to a consumer different colored absorbent articles in a convenient and cost effective manner.

This object has been achieved by providing a package for absorbent articles, containing at least a first absorbent article having a first color and at least a second absorbent article having a second color, the second color being visually distinct from the first color. Advantageously such package contains not only absorbent articles of at least two different colors but might contain also different types of absorbent articles including pantiliners (including conventional ones and string pantiliners) and/or sanitary napkins (including conventional ones and string ones). Color is provided to these articles by the use of colored materials.

In one embodiment according to the present invention at least one of the first and second absorbent article is an absorbent article which has at least one region which is transparent. Transparency is provided to these articles by the use of transparent materials. Such transparent pantiliners have the advantage that their color does not create a highlight, relative to the color of the undergarment in which they are worn and hence are not easily recognizable due to minimum color difference to the undergarment, especially light colored undergarment.

In a preferred embodiment according to the present invention at least one of the first and second absorbent article is an absorbent article which color is black. In a most preferred embodiment the package comprises at least absorbent articles being black and at least absorbent articles being at least partially transparent. Such package might be particularly appealing for younger women wearing fashionable translucent clothing as well as black clothing. Alternatively in another embodiment of the present invention the package comprises at least absorbent articles being white and at least absorbent articles being black. Black and white undergarments are in percent the most commonly worn undergarments by women when considering all ages. Another preferred embodiment is a package comprising absorbent articles which have at least one region of transparency, absorbent articles being black and absorbent articles being white.

Background art of the invention

Packages containing absorbent articles, namely diapers, having different absorbent capacities are known from US 5, 897, 542.

US 5827 251, US 5 986 165 and US 5 964 741 disclose feminine sanitary protection packages comprising a vaginal insertion device, a panty liner and a pouch positioned around the vaginal insertion device and pantiliner.

However no prior art was found on packages comprising a plurality of separated absorbent articles of different colors able to match the colors of desired undergarments.

Summary of the Invention

The present invention relates to a package for a plurality of absorbent articles, containing at least a first absorbent article having a first color and at least a second absorbent article having a second color, the second color being visually distinct from the first color.

Brief description of the drawings

The invention is further described with reference to the accompanying drawing.

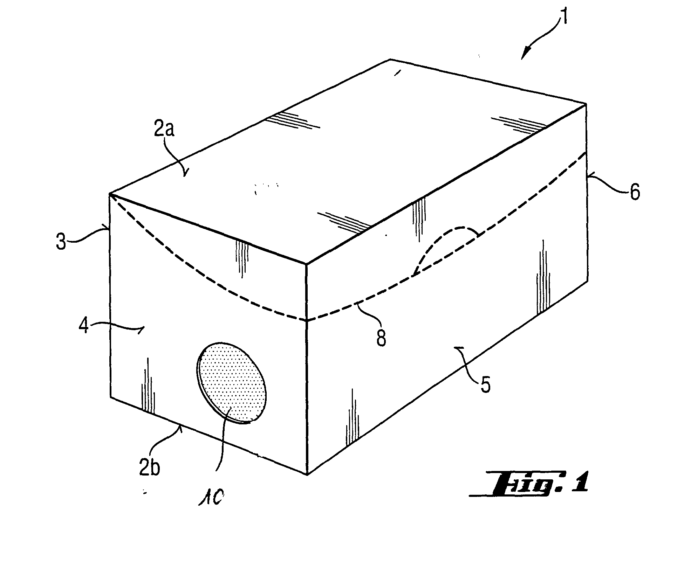

Figure 1 is a general perspective view of a package according to the present invention.

Detailed Description of the Invention

Definitions

"Absorbent articles" as referred to herein are primarily sanitary napkins, pantiliners, or incontinence pads which are worn in the crotch region of a undergarment. However, articles such as sweat-absorbent underarm pads, nursing pads, or collar inserts can also benefit from the present invention. It is even conceivable that baby diapers, adult incontinence diapers, and human waste management devices benefit from the present invention even though they are conventionally not worn in conjunction with an undergarment.

The term 'disposable' is used herein to describe absorbent articles which are not intended to be launched or otherwise restored or reused as absorbent articles (i.e., they are intended to be discarded after a single use and, preferably to be recycled, composted or otherwise disposed of in an environmentally compatible manner).

As used herein the term "wearer-facing" surface refers to the surface of the component of an absorbent article generally oriented to face the wearer during use of the absorbent article. As used herein the term "garment" surface refers to the opposite surface than the wearer facing surface, i.e., to the component of the absorbent article generally oriented to face the garment during use of the absorbent article.

'Color' as referred to herein include any primary/basic colors, e.g., white, black, red, blue, yellow, green, orange, violet, as well as skin color and any declination of the basic colors or mixture thereof. The package herein comprises a plurality of separated absorbent articles of at least two colors visually distinct from one another, i.e., which can be easily recognizable by visual inspection. Color difference between two absorbent articles might also be evaluated by using a colorimeter like for instance Colorimeter Minolta mode CR-300® instrument. Any color is identified by a unique $\Delta E$ value (cf. hereinafter). Typically the difference in color expressed as $\Delta E$ between different absorbent articles packaged in the package according to the present invention is of at least 5, preferably at least 50 and more preferably at least 90.

"Transparent" as used herein refers to the ability of a material or combination of materials to transmit visible light through the body of the material. It is recognized that any material will remove a certain fraction of light and therefore complete transparency cannot exist. Therefore the requirement for transparency according to the present invention is that a color be visually recognizable when viewed by the human eye through the transparent region of the article. There are many various possibilities to measure transparency, one of which is identified hereinafter. According to the present invention said region of transparency should have a transparency value of at least 20%, preferably at least 30%, most preferably at least 40% (ΔE verses reference).

The package

The package according to the present invention comprises a plurality of disposable absorbent articles having different colors. The package according to the present invention typically comprises only one type of absorbent articles provided they are present in at least two different colors or more, or several types of absorbent articles, like different types of feminine protection articles including pantiliners and/or sanitary napkins for conventional undergarments and/ or for string undergarments, provided at least two different colors or more are present. Indeed where at least two types of absorbent articles are present, the first type has at least one color and the second type has at least another color, or at least one type is present in at least two different colors. The package might also comprise absorbent articles (of one type or different types) of different absorbencies (for example sanitary napkins for daily use and night use or heavy fluid occurrence or low flow occurrence).

Typically the package herein comprises from 2 to 100 separated absorbent articles, preferably from 5 to 40 and more preferably from 12 to 35. In the embodiment herein wherein the package contains first and second absorbent articles as defined herein, the numbers of the first absorbent articles to the numbers of the second absorbent articles are in a ratio of 1:10 to 10:1, preferably around 1:1.

It is understood herein that the package might comprise more than two distinct colored absorbent articles. Typically the package might comprise absorbent articles of 4, 5, 6 and more different colors, visually distinct from one another. For example the package can comprise absorbent articles of respectively 7 different colors, thereby given a woman the possibility to wear every day on a weekly basis another colored undergarment so that the colored absorbent article match the color of the undergarment.

Packages for absorbent articles according to the present invention include those constructed as cartons and/ or flexible packages, such as pouches and bags. Standard materials used to construct packages, include but are not limited to paperboard, polymeric film, such as polypropylene films, polyethylene films, coextruded polyethylene and ethylene vinyl acetate films and the like, and coated paper. The package is formed through manipulation of a single sheet of material, such as folding, folding and sealing portions, by adhering multiple sheets to one another or a combination thereof. The package is sealed or adhered by means known in the art, such as heat seal, ultrasonics, adhesives, hook and loop fasteners and the like. Preferably, paperboard and adhesives are used to construct a carton package according to the present invention.

Packaging systems for commodity products are well known in the art. US patent number 3,554,434, US 4,246,286, US 4,782,951, and US 5,694,746, all of which are incorporated by reference herein, disclose a number of paperboard and flexible materials packages and methods of making of the same.

The package can optionally have opening and closure means to enable a user to easily retrieve individual absorbent articles as needed and then close the package to keep the absorbent articles clean and discreetly contained. The opening and closure means can include, but are not limited to flaps activated by applying force to lines of weakening, pursing systems, such as with string, pressure sensitive adhesives, hot melt adhesives, hook and loop fasteners, tab and slit, and interlocking rib and groove strips. Preferably a paperboard carton with a tab and slit closure means is employed according to the present invention.

The absorbent articles can be packaged directly within the package as described herein or they may be individually folded and wrapper within a pouch, an example of which is disclosed in US 4, 556, 146.

An example of suitable package for use herein is illustrated in Figure 1. Package 1 generally has the shape of a parallelepiped box including top and bottom walls 2a, 2b as well as side walls designated 3 to 6 jointly defining a chamber for receiving the absorbent articles therein. A tear line 8 is preferably provided extending through one or more of the side walls 3 to 6 in the vicinity of the top wall 2a. Tear line 8 enables the upper portions of package 1 to be at least partly detached from the rest of package 1 in order to form an upper cover or lid adapted to be lifted or removed in order to access the absorbent articles (not shown) contained in package 1.

The invention is adapted to be applied to packages of any shapes and dimensions, irrespective of whether those packages are provided with parts intended to form covers or lids and/or irrespective of the presence of opening means such as tear line 8. In the presently preferred embodiment, package 1 is comprised of a sheet-like material such as thin cardboard. More preferably package 1 is assembled starting from a single blank of captioned sheet-like material. Optionally the package can be provided with means to identify the color of the absorbent articles contained in the package.

Thus in one broadest aspect the present invention also encompasses a package for absorbent articles containing absorbent articles therein and a means for indicating the color of the articles, whereby the user can determine the color of the undergarments to wear with such absorbent articles. This invention is adapted to be applied to packages of any shapes and dimensions, irrespective of whether those packages contain absorbent articles of different colors. In other words, it is directed to packages containing at least one absorbent article of a given color typically other than white. The means for indicating the color of the article is preferably integral with the outwardly disposed surface of the package.

Advantageously this overcomes the problem of the consumer having to guess which absorbent articles (what color of the absorbent articles) are inside the package that would otherwise only become available to her once purchased and removed. Such package overcomes the need for a package for absorbent articles to distinctly point out the actual color of the product contained therein, so that the woman can choose a product that accurately fits her needs of discretion depending on the color of her undergarment and even clothing (particularly in the case where translucent clothing are worn). Indeed a package is provided comprising a means for determining the color of the absorbent article (preferably pantiliner and/or sanitary napkin), without opening the package and exposing the article contained therein.

Any means known to those skilled in the art for this purpose might be used herein. This means might be any color printing on the package.

This means might be a tab being integral with the package, the tab having any dimension and geometry, provided it indicates the color of the absorbent articles contained in the package. In one embodiment the tab has the dimension and geometry of the absorbent articles contained in the package. The tab can be formed at the same time the package is formed, or can be alternatively be formed separately and attached to the package. The tab can be constructed from similar or dissimilar materials to that of the package. Preferably the tab is attached to the package at a single point, thereby allowing a user to partially dissociate the color representation from the package by rotating the tab away from the package.

This means might also be a sheet of the color of interest, which is releasably attached to one or more outwardly disposed surface of the package. The sheet might have any dimension and geometry including those corresponding to that of the absorbent article contained in the package. Typically the area of the package that is covered by the sheet is transparent so that when the sheet is at least partially removed, the absorbent articles contained in the package can be visualized. The visualization allows a user to see the color of the actual absorbent articles contained in the package, compared to that of the color rendition as typically shown on the outer surface of the package, while maintaining the discreteness if desired by either not removing or refastening the sheet. The sheet is preferably attached by pressure sensitive adhesive and can be refastened if removed

This means preferably simply is a transparent area or windows 10 (see Figure 1) which can be transparent so that the colors of the contained absorbent articles can be visualized. Typically the package is provided with only one means (e.g., one window, one colored printed area or tab or sheet) indicating the color(s) of the absorbent articles contained therein. If absorbent articles of different colors are contained in the package, the package might also be provided with several means (e.g., several windows) each of them indicating one color of the different colored absorbent articles contained in the package.

The absorbent articles packaged in the package according to the present invention

The absorbent articles packaged in the package according to the present invention have a wearer facing surface directed towards the wearer during use (typically the outer surface of the topsheet) and a garment facing surface directed towards garment/undergarment during use (typically the outer surface of the backsheet). In a preferred embodiment the wearer facing surface is provided by the topsheet of the absorbent article and the garment facing surface is provided by the backsheet of the article, as per examples of the invention illustrated herein after. Typically absorbent articles are conventionally constructed of three main elements: The topsheet, facing the user of the article during use and being liquid pervious in order to allow liquids to pass into the article; the backsheet, providing liquid containment such that absorbed liquid does not leak through the article, this backsheet conventionally provides the garment facing surface of the article; and the absorbent core sandwiched between the topsheet and the backsheet and providing the absorbent capacity of the article to acquire and retain liquid which has entered the article through the topsheet.

Many absorbent articles and constructions, including particular materials, are known in the art and have been described in ample detail over time. All of such materials are useful in the context of the present invention, provided they are available in the desired color (e.g., black, gray, green, red and so on) or provided that they allow a minimum degree of transparency to be achieved for the absorbent articles having at least one region of transparency. Typically this will require only moderate modification of the material composition while maintaining the majority of the conventional material characteristics. In the following, examples of materials which are particularly beneficial for the use in either colored absorbent articles (e.g., black and so on) or in transparent absorbent articles are mentioned. Those skilled in the art Will readily be able to identify alternative materials which can also be used herein.

It is understood herein that by absorbent article of a given color, it includes an absorbent article having at least its wearer facing surface or at least its garment facing surface of that given color. Garment/undergarment facing surface matching the color of the undergarment is desired for discretion in use especially when wearing transparent clothing or colored clothing. Wearer facing surface matching the color of the undergarment is desired for discretion in more intimate moments of women life, for example to minimize or even avoid visual recognition of the presence of an absorbent article when removing undergarment.

Preferably both the wearer facing surface typically provided by a topsheet and the garment facing surface typically provided by a backsheet have the same color. The absorbent core might preferably also have the same color as the wearer facing surface to minimize recognition of it, thereby providing a masking effect of the body fluid in use. This masking effect is especially desired for pantiliner usage where the fluid discharge (vaginal fluid and/or urine discharge) is relatively limited and the wearer has an habit of wearing pantiliners for identical periods of time over subsequent days (e.g., one or two pantiliners per day)

Topsheet

The topsheet if present is compliant, soft feeling, and non-irritating to the wearer's skin. The topsheet also can have elastic characteristics allowing it to stretch in one or two directions. Further, the topsheet is fluid pervious permitting fluids (e.g., menses and/or urine) to readily penetrate through its thickness. A suitable topsheet can be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers.

Preferred topsheets for use for the colored absorbent articles of the present invention are selected from high loft nonwoven topsheets and aperture formed film topsheets. At least in the region where liquid is expected to be discharged onto the absorbent article the apertured formed films are preferred because they are pervious to body exudates and yet non-absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135 (Thompson), issued December 30, 1975; U.S. Patent 4,324,246 (Mullane, et al.), issued April 13, 1982; U.S. Patent 4,342,314 (Radel. et al.), issued August 3, 1982; U.S. Patent 4,463,045 (Ahr et al.), issued July 31, 1984; and U.S. 5,006,394 (Baird), issued April 9, 1991. Particularly preferred microapertured formed film topsheets are disclosed in U.S. patent 4,609,518 (Curro et al), issue September 2, 1986 and U.S. patent 4,629,643 (Curro et al), issued December 16, 1986.

It is, however, particularly preferred that the topsheet be a so-called hybrid topsheet in which the wearer contacting surface is provided in its longitudinal center by an apertured formed film while a region not including the center is provided with a non-woven such as e.g. the high loft non-woven mentioned above or other non-woven which does provide particularly skin friendliness. Such topsheets have been disclosed in EPA-523 683, EP-A-523 719, EP-A-612 233, or EP-A-766 953.

Conventionally such topsheet have been provided with a coloring material such as titanium dioxide to provide a white opacity. Alternatively other coloring materials might be used to provide other colors like black, gray, red , yellow, green, blue, orange, violet and the like and mixtures thereof.

Colored topsheet can typically be provided made of any of the materials mentioned above by different methods well known to those skilled in the art, including pigmenting the materials, dying the materials or color printing the materials.

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer through the topsheet faster than if the body surface was not hydrophilic. In a preferred embodiment, surfactant is incorporated into the polymeric materials of the formed film topsheet such as is described in PCT Publication No. WO93/09741, "Absorbent Article Having A Nonwoven and Apertured Film Coversheet" filed on November 19, 1991 by Aziz, et al. Alternatively, the body surface of the topsheet can be made hydrophilic by treating it with a surfactant such as is described in the above referenced U.S. 4,950,254. The surfactant chosen should not affect the color of the materials

In one embodiment the package of the present invention comprises absorbent articles with at least one region of transparency. Transparency is typically obtained by using transparent materials. Simply omitting the coloring material from the polymer provides a transparent film or nonwoven which has a high degree of light transmission. The absence of the color filler, such as titanium dioxide, however does not cause any substantial material changes. In fact, it has been mentioned in the art that the desire for a particular white material may cause a polymeric material to have undesirable physical characteristics such as brittleness which have led to a body of art according to which multi-layered films are created in which only some of the layers are provided with a color filler while other layers provide structural stability and integrity but do not comprise the color material. Hence the absence of a color filler in the polymeric material from which the topsheet can be made also provides the additional benefit of better material characteristics (besides less costs and a reduced environment burden). Alternatively, the topsheet may be provided with non white pigmented fillers which impart a tint to the topsheet in a desirable color such as black, red, yellow, blue, black and green whilst still ensuring the desired transparency. The region of the topsheet which is transparent preferably contains not more than 1% by weight of said topsheet (including multilayers) of white fillers, preferably not more than 0.5% by weight of white fillers and is most preferably free of white fillers. In embodiments containing non white pigmented fillers, the topsheet typically comprises from 0.1% to 3%, preferably from 0.3% to 0.6% of said filler. An example of non white pigmented fillers includes green 7-(74260) CAS 1328753-6 . The topsheet can be completely transparent or can be provided only with regions of transparency. Preferably the topsheet is at least transparent in the region where it extends beyond the absorbent core such that the region of the absorbent article extending beyond the periphery of the absorbent core can be provided with transparency.

Backsheet

In general the backsheet is compliant, flexible and soft feeling. The backsheet prevents the exudes absorbed and contained in the absorbent core from wetting clothes that contact the absorbent article such as an undergarments. Preferably the backsheet is impervious to liquids (e.g., menses, sweat and/or urine). It can be manufactured from a thin plastic film, although other flexible liquid impervious materials can also be used. As used herein, the term "flexible" refers to materials that are compliant and will readily conform to the general shape and contours of the human body. The backsheet preferably also can have elastic characteristics allowing it to stretch in one or two directions.

The backsheet can comprise a woven or nonwoven material, polymeric films such as thermoplastic films of polyethylene or polypropylene, or composite materials such as a film-coated nonwoven material or fiber coated film. Conventionally absorbent articles comprise a backsheet of a polyethylene film having a thickness of from about 0.012 mm to about 0.051 mm.

The backsheet is preferably breathable, i.e. allows the transmission of water vapor, or even more preferable the transmission of air, however without sacrificing its main purpose to provide leakage protection for absorbed liquids. The backsheet can also comprise more than one breathable layer so as to replace a single breathable backsheet layer by at least 2 or 3 layers of a different or the same material. In particular two breathable layers forming together the breathable backsheet are preferred.

According to the present invention backsheets made of any material for example backsheet made from polymeric materials can be provided with a desired color by using conventional methods known to those skilled in the art for this purpose, including dying, pigmenting and/or printing techniques.

As for the topsheet the entire backsheet can be provided with the same color or with different colors. In a preferred embodiment the topsheet and backsheet are made of the same color to provide enhanced discretion in all usage conditions.

In the embodiment herein wherein the absorbent article has at least one region of transparency, the backsheet is typically made of a polyethylene. Actually any backsheet made from polymeric material can be provided with transparency by eliminating the white color filler, which conventionally was, for white materials, titanium dioxide. As will be recognized by those skilled in the art the transparency of the backsheet can be provided in the same fashion as in the topsheet and may comprise non white pigmented fillers. As with the topsheet the entire backsheet can be completely transparent or it may also only be provided with regions of transparency. Preferably, the region of transparency of the backsheet corresponds to the transparency region provided in the topsheet as otherwise the transparency of the overall product would suffer. Therefore the transparency of the backsheet is preferably provided at least in the region in which the backsheet extends beyond the periphery of the absorbent core, which is the region where the backsheet conventionally is joined to the topsheet. In one embodiment the topsheet and the backsheet are completely transparent over their entire surfaces. This not only provides the previously mentioned benefits of a transparent peripheral edge of the product, but also allows the wearer of such a product to be able to observe the core and the absorbent liquid contained therein. As a result the wearer can readily identify if the absorbent capacity of the product has been reached and accordingly decide on when to replace the absorbent article with a new one for self comfort and cleanness.

Absorbent core

Conventionally the absorbent core can be a single entity or comprise several layers. It can includes the following components: (a) optionally a primary fluid distribution layer; (b) optionally a secondary fluid distribution layer;

(c) a fluid storage layer; (d) optionally a fibrous layer underlying the storage layer; and (e) other optional components.

### a. Optional Primary Fluid Distribution Layer

One optional component of the absorbent core according to the present invention is the primary fluid distribution layer. This primary distribution layer typically underlies the topsheet (if present) and is in fluid communication therewith. The primary distribution layer acquires body fluid for ultimate distribution to the storage layer. This transfer of fluid through the primary distribution layer occurs mainly in the thickness, but may also provide distribution along the longitudinal and transverse directions of the article.

### b. Optional Secondary Fluid Distribution Layer

Also optional according to the present invention is a secondary fluid distribution layer. This secondary distribution layer typically underlies the primary distribution layer and is in fluid communication therewith. The purpose of this secondary distribution layer is to readily acquire bodily fluid from the primary distribution layer and distribute it along the longitudinal and transverse directions of the article before transfer to the underlying storage layer. This helps the fluid capacity of the underlying storage layer to be fully utilized.

### c. Fluid Storage Layer

Positioned in fluid communication with, and typically underlying the primary or secondary distribution layers, is a fluid storage layer. It comprises preferably super-absorbent or gelling materials usually referred to as "hydrogels," "superabsorbent", "hydrocolloid" materials. Absorbent gelling materials are those materials that, upon contact with aqueous fluids, especially body fluids, imbibe such fluids and thus form hydrogels. These absorbent gelling materials are typically capable of absorbing large quantities of aqueous body fluids, and are further capable of retaining such absorbed fluids under moderate pressures. In the prior art these absorbent gelling materials are typically in a granular form of discrete, non-fibrous particles. However, these super-absorbent gelling materials can also be provided in non-granular form, typically in a fibrous form.

In the fluid storage layer these absorbent gelling materials can be dispersed homogeneously or non-homogeneously in a suitable fibrous matrix also referred to as carrier. Suitable carriers include cellulose fibers, in the form of fluff, such as is conventionally utilized in absorbent cores. Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferred synthetic and man-made fibers have a denier of from about 3 denier per filament to about 25 denier per filament, more preferably from about 5 denier per filament to about 16 denier per filament. Also preferably, the fiber surfaces are hydrophilic or are treated to be hydrophilic. A non transparent storage layer can also include filler materials, such as Perlite, diatomaceous earth, Vermiculite, etc., that lower rewet problems. Further the storage layer may comprise a binder including but not limited to Latex binders which can be sprayed as an aqueous solution onto the surface of the storage layer prior to curing. If transparency or given color is required, these carrier materials must be selected to provide respectively the desired transparency or desired color.

If the absorbent gelling materials are dispersed non-homogeneously in a fibrous matrix, the storage layer can be locally homogeneous, i.e. have a distribution gradient in one or several directions within the dimensions of the storage layer. Non-homogeneous distribution thus includes e.g. laminates of the fibrous carriers enclosing the absorbent gelling materials.

Typically, the storage layer comprises from 5% to 95% absorbent gelling materials, preferably from 5% to 50%, most preferably from 8% to 35%, absorbent gelling materials. Further the storage layer can comprise from 5% to 95% carrier fibers, preferably from 95% to 50%, most preferably from 92% to 65% carrier fibers.

Suitable absorbent gelling materials for use herein will most often comprise a substantially water-insoluble, slightly crosslinked, partially neutralized, polymeric gelling material. This material forms a hydrogel upon contact with water. Such polymer materials can be prepared from polymerizable, unsaturated, acidcontaining monomers. Suitable unsaturated acidic monomers for use in preparing the polymeric absorbent gelling material used in this invention include those listed in U.S. Patent 4,654,039 (Brandt et al), issued March 31, 1987, and reissued as RE 32,649 on April 19, 1988. Preferred monomers include acrylic acid, methacrylic acid, and 2-acrylamido-2-methyl propane sulfonic acid. Acrylic acid itself is especially preferred for preparation of the super-absorbent material in the embodiment herein where a transparent core is desired, as it also has a 'natural' transparency which is not optimal but acceptable if the desired transparency is not too high.

Whatever the nature of the basic polymer components of the hydrogel-forming polymeric absorbent gelling materials, such materials will in general be slightly crosslinked. Crosslinking serves to render the hydrogel-forming polymer gelling materials substantially water-insoluble, and cross-linking thus in part determines the gel volume and extractable polymer characteristics of the hydrogels formed from these polymeric gelling materials. Suitable crosslinking agents are well known in the art and include, for example, those described in greater detail in U.S. Patent 4,076,663 (Masuda et al), issued February 28, 1978. Preferred crosslinking agents are the di- or polyesters of unsaturated mono- or polycarboxylic acids with polyols, the bisacrylamides and the di- or triallyl amines. Other preferred crosslinking agents are N,N'-methylenebisacrylamide, trimethylol propane triacrylate and triallyl amine. The crosslinking agent can generally constitute from about 0.001 mole percent to 5 mole percent of the resulting hydrogel-forming polymer material. More preferably, the crosslinking agent will constitute from about 0.01 mole percent to 3 mole percent of the hydrogel-forming polymeric gelling material.

The slightly crosslinked, hydrogel-forming polymeric gelling materials are generally employed in their partially neutralized form. For purposes of the present invention, such materials are considered partially neutralized when at least 25 mole per-cent, and preferably at least 50 mole percent of monomers used to form the polymer are acid group-containing monomers that have been neutralized with a salt-forming cation. Suitable salt-forming cations include alkali metal, ammonium, substituted ammonium and amines. This percentage of the total monomers utilized which are neutralized acid group-containing monomers is referred to herein as the "degree of neutralization."

While these absorbent gelling materials can typically be used in granular form, it is also possible to use them in a non-granular form for example as macrostructures such as fibers, sheets or strips. These macrostructures can be prepared by forming the particulate absorbent gelling material into an aggregate, treating the aggregated material with a suitable crosslinking agent, compacting the treated aggregate to densify it and form a coherent mass, and then curing the compacted aggregate to cause the crosslinking agent to react with the particulate absorbent gelling material to form a composite, porous absorbent macrostructure. Such porous, absorbent macrostructures are disclosed, for example, in U.S. Patent 5,102,597 (Roe et al), issued April 7, 1992.

Colored absorbent core can be obtained by any method known to those skilled in the art for coloring materials, including pigmenting, printing and dying.

If the absorbent core is desired to be transparent then it can be provided by a layer comprising 100% of an absorbing gelling material. Such gelling material must then be transparent, typical example of transparent absorbent gelling material is a water based hydrogel adhesive which is not saturated with water. Such hydrogel adhesives are known as body adhesives but can be used in a less water saturated form as transparent absorbent cores. An example of such a material is Hydrmelt NP-2257, available from the H.B. Fuller Company, Lüneburg, Germany. Such materials are particularly advantageous in that they also function as a construction adhesive for the article and do not necessitate the addition of construction adhesives between the core, topsheet and backsheet. Alternatively the core can be provided from a transparent fibrous superabsorbent nonwoven.

According to a particularly preferred embodiment of the present invention the absorbent core comprises only one layer, preferably one fluid storage layer.

d. Optional Fibrous Layer

An optional component for inclusion in the absorbent cores according to the present invention is a fibrous layer adjacent to, and typically underlying the storage layer. This underlying fibrous layer would typically provide the same function as the secondary fluid distribution layer.

e. Other Optional Components

The absorbent cores can include other optional components normally present in absorbent webs. For example, a reinforcing scrim can be positioned within the respective layers, or between the respective layers, of the absorbent cores. Such reinforcing scrims should be of such configuration as to not form interfacial barriers to fluid transfer, especially if positioned between the respective layers of the absorbent core. Given the structural integrity that usually occurs as a result of thermal bonding, reinforcing scrims are usually not required for the absorbent structures herein.

More generally the absorbent core of the absorbent articles used herein may be of any color including black, white, or may be provided with a region of transparency depending on the absorbent articles it will be included. In cases where the transparent region of the absorbent article is outside the region co-extensive with the absorbent core, then the absorbent core need not be transparent and can be provided in any conventional fashion known. This is particularly preferable for absorbent articles in which only those parts of the absorbent article which are not co-extensive with the absorbent core are desired to be transparent, for example in an embodiment where a sanitary napkin is provided with transparent wings or a transparent peripheral region.

In the alternative if all or part of the region of the article co-extensive with the absorbent core is desired to be transparent then of course the absorbent core itself must support this transparency and materials such as polymeric fibers or superabsorbent materials such as hydrogels which are conventionally and frequently used, need to support this transparency by being provided in a transparent form, i.e. without a white color filler such as titanium dioxide as has been explained above for the topsheet and the backsheet already, for non-white pigmented colors. Where materials for the absorbent core are used which cannot be altered to be transparent it is then necessary to replace these materials by transparent materials having otherwise similar characteristics.

Thus in another embodiment the transparent absorbent articles suitable for use in the package herein have the topsheet, backsheet and core which are completely transparent over their entire surfaces. The advantage of this embodiment is that it has been surprisingly found that not only is the product not recognizable when worn in combination with colored undergarments and or semitranslucent garments, the contour of the article itself under the garment is no longer discernable. This is especially useful when the articles are worn in combination with tight fitting clothing.

Another component which can be included in the absorbent core of the absorbent articles herein and preferably is provided close to or as part of the primary or secondary fluid distribution layer are odor control agents. Typically active carbon coated with or in addition to other odor control agents, in particular suitable zeolite, silica or clay materials, are optionally incorporated in the absorbent core.

Active carbon is the preferred odor control agent for use in the dark colored absorbent articles like black one, as this material provides outstanding odor control properties at low cost.

Physical characteristics of absorbent cores

Absorbent cores are usually non extensible and non-elastic, however, they can be rendered extensible and depending on the selected materials can also be made to have elastic characteristics. The term "extensible" as used hereinafter refers to a structure which under external forces such as those occurring during use extends in the direction of the forces or in the direction of a component of the forces in cases where only mono directional extensibility is provided.

The term "elastic" as used hereinafter refers to extensible structures which return at least partially to their initial state after the forces causing the extension cease to be exerted. Absorbent cores can be corrugated or pleated in one or several directions to provide a certain extensibility while selection of elastic fibers for the structure can provide elasticity.

The absorbent cores should preferably be thin. A thickness of less than 5 mm, preferably less than 3 mm, more preferably less than 1.8 mm, and even more preferable between 0.1 and 1.8 mm is desirable such that the resulting articles can also have a low thickness.

In the context of transparency, thinness of the transparent region of the article is also of key importance since the thicker a material is the less transparent it will be.

Absorbent article construction

Absorbent articles for use herein are constructed as described in the art. In the embodiment herein wherein the absorbent article has at least one region of transparency or is transparent the conventional means for joining portions of material together must be carefully considered to ensure that the objective of creating a transparent region is not lost. E.g. the adhesive used to join the topsheet to the backsheet in the region outside the absorbent core should either be transparent or it should be eliminated and be replaced by e.g. crimping.

Transparent adhesives are widely available e.g. conventional adhesives can be transparent and many polymer based adhesives are transparent since they have never been used for their opacity and hence their compositions did never include color fillers to start with.

Absorbent article design

The color and transparency as indicated above can be used beneficially in the context of sanitary napkins, panty liners and sweat pads (underarm or collar). A new product design, which is a sub-form of a sanitary napkin or panty liner form, namely thong shaped sanitary napkins or panty liners, so called thong liners or string pantiliners, are particularly susceptible to the present invention. The thong liner design is such that it provides the sanitary napkin or panty liner with a shape such that it can be worn in thong slips, G-string undergarments or string panties, hence the thong shape is fundamentally triangular or trapezoidal.

Optional components of the absorbent articles

Optionally, the absorbent articles herein can comprise all those components typical for the intended product use. For example absorbent articles can comprise components such as wings in order to improve their positioning and soiling protection performance especially towards the rear end of the article. Such designs are shown for example in EP 130 848 or EP 134 086, Thong liners with wings are shown in US design 394,503, UK designs 2,076,491 and 2,087,071 as well as internationally filed industrial model DM 045544, filed under the Hague Agreement, registered on October 21, 1998.

Where the absorbent articles comprises at least one region of transparency, the wings, if present can be the region of the absorbent article which comprises the transparent region. In fact an article design in which the wings are transparent is highly desirable, particularly for thong liners, it can be desirable to have transparent wings. The reason is that wings on thong liners extend substantially to the rear end of the article and when folded onto the external side of a thong slip are rather easily visible.

Irrespective whether the wings are specially designed for thong liners or for conventional absorbent articles they can be provided as separate pieces and be attached to the thong liner or they can be integral with the materials of the thong liner, e.g. by being integral extension of the topsheet, the backsheet or a combination thereof. If the wings are attached then they can be attached in a basic outward pointing position or already be predisposed towards their in-use position, i.e. towards the longitudinal centerline. If the wings are integral extensions of the topsheet or the backsheet or both then they are provided with the same transparency as these materials.

Where the absorbent article has a given color, the wings if present are colored to match the undergarment color.

In its broadest aspect the present invention is also directed to absorbent articles like pantiliners or sanitary napkins provided with wings wherein the wings have a color which match the color of a given undergarment, the wing color being either visually distinct from the remaining of the absorbent article or of the same color. This can be provided in execution where the wings are an integral extension of the topsheet and/or backsheet as well as in execution where they are provided as separate pieces. Any convention material might be used for this purpose, provided they are colored as desired. Thus the present invention also encompasses an absorbent article wherein said article is a sanitary napkin or a pantiliner for use in the crotch region of an undergarment said absorbent article comprising a pair of wings intended for folding around the crotch portion of an undergarment and wherein each of said wings are provided with a color visually distinct from the remaining of the absorbent article, the color of said wing matching the color of the desired undergarment. For examples hygienic white absorbent articles (pantiliner or sanitary napkin) with black wings.

Most preferred absorbent articles will comprise a fastening adhesive for attachment. The design of the fastening adhesive must be selected such that it does not interfere with the desired transparency but transparent adhesives will ensure that. In the case of sanitary napkins, pantiliners or thong liners a so called panty fastening adhesive is preferred to be present on the backsheet for attachment to an undergarment. However, for sweat pads, e.g. underarm sweat pads, either attachment to an adjacent garment or attachment to the skin of the wearer directly can also be considered. Of course, such direct skin attachment, which is conventionally provided by water gel, hydrogel or oil gel based body adhesives, can also be used in sanitary napkins or body liners (in contrast to pantiliners).

Transparent Evaluation

General Definition

Optically transparent: Permitting the passage of light radiation.

Optically transparent medium: A medium which has the property of transmitting rays of lights in such a way that the human eye may see through the medium distinctly.

In general transparency is the ability of a material to transmit light through itself and consequently by the transparency it is possible to see object/colors/ printed or written text through such material.

Transparent Product

Accordingly to the above terminology a transparent region of a product is defined by:

1. having the property of transmitting rays of light in such a way that written or printed text/characters and colors located opposite the transparent product can be clearly viewed by the human eye. and/or

2. having the property of transmitting rays of light in such a way that the human eye may see through the product.

One of the main advantages delivered by a transparent product is that its presence (color) is less recognizable by the human eye, such that, primarily, the color of the undergarment (or other clothing) is recognizable (visual discreteness, no product awareness).

Colors can be measured according an internationally recognised 3D solid diagram of colors where all colors that are perceived by the human eye are converted into a numerical code. This system is based on three dimensions (x,y,z) and specifically L*, a*, b*.

When a color is defined according to this system L* represents lightness (0 = black, 100 = white), a* and b* independently each represent a two color axis, a* representing the axis red/green (+a = red, -a = green), while b* represents the axis yellow/blue (+b = yellow, -b = blue).

Any color is identified by a unique ΔE value which is mathematically expressed by the equation:

$$\Delta E = [\, (L)^2 + (a)^2 + (b)^2)]^{1/2}$$

ΔE represents graphically the distance between the reference color and the no color point (i.e. centre of sphere L = 50, a = 0, b = 0) of the 3d model.

The ability to see a color through a material (or product) is measured as an index of transparency. If a material is 100% transparent, it is possible to measure the same ΔE value for the above color alone and through the material. As a reference the color white is used. The closer the color is to the white reference (when both are viewed through the material/product) the less transparent that material (or product) will be considered.

Color Transparency Methodology

Color can measured using the colorimeter MINOLTA mode CR-300 instrument (available from the Minolta Company, Japan) which provides the coordinates L*, a*, b* and from which the ΔE value can be determined.

The standard colors used in this measurement are the primary colors Cyan, Magenta and Yellow references of PANTONE Color Specifier 747XR and the white calibration reference plate of the colorimeter instrument.

The color grade coordinate values for the material to be tested for each of the color references Cyan, Magenta and Yellow is determined by placing the material above the color specific reference and taking a reading from the colorimeter and calculating ΔE.

For each of the reference colors (rc) the ΔE value is recalculated by setting the scale such that $\Delta E_{rc}$ result, referred to as $\Delta\Delta E_{rc}$ is zero (i.e. 100% transparent) and that $\Delta E_{white}$ with respect to the reference color is referred to as $\Delta\Delta E_w$ which is 62.2 for example and represents 0% transparency.

This can be represented by the formulae below

$$\Delta\Delta E_{rc} = [\, (L_{rc} - L_{rc})^2 + (a_{rc} - a_{rc})^2 + (b_{rc} - b_{rc})^2]^{1/2} = 0$$

$$\Delta\Delta E_w = [\, (L_{rc} - L_w)^2 + (a_{rc}\, a_w)^2 + (b_{rc} - b_w)^2]^{1/2}$$

The ΔΔE value for the product/transparent region of the product for each reference color ($\Delta\Delta E_p$) is then calculated using the following formula:

$$\Delta\Delta E_p/_{rc} = [\, (L_{rc} - L_p)^2 + (a_{rc} - a_p)^2 + (b_{rc} - b_p)^2]^{1/2}$$

Transparency of the product for each reference color is determined according to the formula:

$$\text{Transparency} = 100 - \frac{\Delta\Delta E_{rc} \times 100}{\Delta\Delta E_p}$$

The total transparency is the average value of the transparency for each reference color i.e.

$$\text{Total transparency} =$$

$$100 \; \frac{\text{Transparency}_{cyan} + \text{Transparency}_{magenta} + \text{Transparency}_{yellow}}{3}$$

Examples

Examples of pantiliners to be packaged in a package (Figure 1) according to the present invention are as following:

Option 1

The pantiliner comprises a transparent apertured polyethylene formed film topsheet (transparent CPM DH® available from BP Chemicals), a spiral layer of adhesive (PM17®, available from Savare), an adhesive hydrogel absorbent core (AGM glue code NP-2257, basis weight 180gsm, available from Fuller), a nonwoven backsheet without pigment (code W16Fio, basis weight 16gsm, available from BBA Corovin), stripes of panty fastening adhesive (HL1461® available from Fuller) and release paper.

Option 2

The pantiliner comprises a transparent apertured polyethylene formed film topsheet (transparent CPM DH® available from BP Chemicals, Wasserburg-Germany), spiral layer of adhesive (PM17®, available from Savare), a nonwoven thermal bonded polypropylene core (polypropylene and super absorbent fibres, code NPS80, basis weight 80 gsm, available from BFF, UK), spiral layer of adhesive (PM17®, available from Savare), a nonwoven backsheet without pigment (code W16Fio, basis weight 16gsm, available from BBA Corovin, Peine-Germany), stripes of panty fastening adhesive (HL1461, available from Fuller, Luneburg-Germany) and release paper.

Option 3

The pantiliner comprises a black nonwoven spunbonded polyethylene SBPE, basis weight 27 gsm (code T27CXC® available from BBA, Linotec) with black apertured polyethylene formed film (black CPM® dual holes available from BP Chemicals, Wasserburg-Germany) as the topsheet, as the secondary topsheet a black nonwoven SBPE (code T27CXC® available from BBA, Linotec), then a spiral layer of adhesive (H2128® from Ato Findley), a white absorbent core with 10% super absorbent fibers (code GH.100.1004, basis weight 100 gsm, commercially available from Concert GmbH-Falkenhagen Germany), a spiral layer of adhesive (PM17® commercially available from Savare), polyethylene micro embossed film with black pigments as the backsheet (code ST 700®, available from Britton Taco LtD. Winsford Chesire UK), stripes of panty fastening adhesive (HL1461®, available from Fuller, Luneburg-Germany) and a release paper.

Option 4

The pantiliner comprises a white nonwoven spunbonded polyethylene SBPE, basis weight 27 gsm (code T27AXC® available from BBA, Linotec, Germany) with white apertured polyethylene formed film (white CPM® dual holes, code 45015 available from BP Chemicals, Wasserburg-Germany) as the topsheet, a secondary topsheet white nonwoven SBPE (code T27AXC® available from BBA, Linotec), a spiral layer of adhesive (H2128® from Ato Findley), an air laid absorbent laminate (code XO03801002 available from Korma), adhesive (PM17® commercially available from Savare), a white apertured polyethylene formed film as a secondary backsheet (code S225 MD25 commercially available from Tredegar, NL) and a white microporous film backsheet (code XBF112W, commercially available from Exxon, CH), stripes of panty fastening adhesive (NS834-2823, available from National Starch) and a release paper.

According to the present invention a package is provided containing 15 pantiliners according to option 1 described herein before and 15 pantiliners according to option 3 described herein before. Another package is provided comprising 10 pantiliners of option 1,10 pantiliners of option 3 and 10 pantiliner of option 4. Another package is provided comprising 10 pantiliners of option 3 and 20 pantiliners of option 4.

**Claims**

1. A package for absorbent articles, containing at least a first absorbent article having a first color and at least a second absorbent article having a second color, the second color being visually distinct from the first color.

2. A package according to claim 1, wherein said first and second absorbent articles have respectively a garment facing surface and a wearer facing surface and wherein at least the wearer facing surface or garment facing surface of the first absorbent article is of said first color and wherein at least the wearer facing surface or garment facing surface of the second absorbent article is of said second color.

3. A package according to any of the preceding claims wherein said first and second absorbent articles respectively comprise a liquid pervious topsheet, a liquid impervious, but preferably breathable, backsheet and an absorbent core intermediate said topsheet and said backsheet, wherein at least the topsheet and backsheet of the first absorbent article are of the first color and/or at least the topsheet and backsheet of the second absorbent article are of the second color.

4. A package according to claim 3 comprising said first and second absorbent articles, wherein the absorbent core of the first absorbent article matches the color of the topsheet of the first absorbent article and/or wherein the absorbent core of the second absorbent article matches the color of the topsheet of the second absorbent article.

5. A package according to any of the preceding claims wherein the color difference between the first color of the first absorbent article and the second color of the second absorbent article is at least 5, preferably at least 50 and more preferably at least 90, when measured with a Minolta mode CR300® colorimeter.

6. A package according to any of the preceding claims wherein at least the color of the first or second absorbent article is black.

7. A package according to any of the preceding claims 1 to 5, wherein at least the color of the first or second absorbent article is white.

8. A package according to any of the preceding claims, wherein at least the first or second absorbent article is an absorbent article having at least one region of transparency, typically said absorbent article has a topsheet, a backsheet and an absorbent core disposed between the topsheet and backsheet, said topsheet and backsheet extend beyond the periphery of said absorbent core and are joined to each other in the region extending beyond the periphery of said absorbent core, the region of said topsheet and said backsheet extending beyond the periphery of the absorbent core being transparent.

9. A package according to claim 8, wherein said transparency has a transparency value of at least 20%, preferably at least 30% and more preferably at least 40%.

10. A package according to any of the preceding claims wherein said absorbent articles are pantiliners and/or sanitary napkins and preferably are string-pantiliners and/or conventional pantiliners.

11. An absorbent article wherein said article is a sanitary napkin or a pantiliner for use in the crotch region of an undergarment said absorbent article comprising a pair of wings intended for folding around the crotch portion of an undergarment and wherein each of said wings are provided with a color visually distinct from the color of the remaining of the absorbent article, the color of the wings matching the color of the undergarment.

12. An absorbent article according to claim 10, wherein each entire wing is of that color, which is typically visually distinct from white.

13. A package for absorbent articles containing absorbent articles therein and a means for indicating the color of the articles, whereby the user can determine the color of the undergarment to wear with such absorbent articles.

14. A package according to claim 13, wherein the means for indicating the color of the article is integral with the outwardly disposed surface of the package.

**Fig. 1**

| | European Patent Office | **EUROPEAN SEARCH REPORT** | Application Number |
|---|---|---|---|
| | | | EP 00 11 5724 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 97 45088 A (PROCTER & GAMBLE) 4 December 1997 (1997-12-04) * page 22, paragraphs 2,3 * --- | 1,3,13, 14 | A61F15/00 |
| X | WO 00 00147 A (HAEUSER KAI ;HAEUSER ROGER (DE)) 6 January 2000 (2000-01-06) * page 7, paragraph 4 * --- | 1 | |
| A | EP 0 948 949 A (WALLACE CAMERON & COMPANY LIMI) 13 October 1999 (1999-10-13) * column 1, paragraphs 2-4 * --- | 1 | |
| A | US 3 638 651 A (TORR DAVID) 1 February 1972 (1972-02-01) * claims 1,4 * ------ | 11 | |

| | | | TECHNICAL FIELDS SEARCHED (Int.Cl.7) |
|---|---|---|---|
| | | | A61F |

The present search report has been drawn up for all claims

| Place of search | Date of completion of the search | Examiner |
|---|---|---|
| BERLIN | 20 December 2000 | Korth, C-F |

EPO FORM 1503 03.82 (P04C01)

CATEGORY OF CITED DOCUMENTS

X : particularly relevant if taken alone
Y : particularly relevant if combined with another document of the same category
A : technological background
O : non–written disclosure
P : intermediate document

T : theory or principle underlying the invention
E : earlier patent document, but published on, or after the filing date
D : document cited in the application
L : document cited for other reasons

& : member of the same patent family, corresponding document

EP 1 174 104 A1

**ANNEX TO THE EUROPEAN SEARCH REPORT**
**ON EUROPEAN PATENT APPLICATION NO.**

EP 00 11 5724

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2000

| Patent document cited in search report | | Publication date | Patent family member(s) | | | Publication date |
|---|---|---|---|---|---|---|
| WO 9745088 | A | 04-12-1997 | AU | 3150297 | A | 05-01-1998 |
| | | | AU | 3150397 | A | 05-01-1998 |
| | | | BR | 9709610 | A | 10-08-1999 |
| | | | BR | 9709628 | A | 10-08-1999 |
| | | | CA | 2256472 | A | 04-12-1997 |
| | | | CN | 1225572 | A | 11-08-1999 |
| | | | CZ | 9803928 | A | 14-04-1999 |
| | | | EP | 0901361 | A | 17-03-1999 |
| | | | JP | 2000508215 | T | 04-07-2000 |
| | | | JP | 11513916 | T | 30-11-1999 |
| | | | NO | 985591 | A | 01-02-1999 |
| | | | WO | 9745089 | A | 04-12-1997 |
| | | | US | 5947302 | A | 07-09-1999 |
| | | | US | 6093027 | A | 25-07-2000 |
| | | | US | 5865322 | A | 02-02-1999 |
| | | | US | 5839585 | A | 24-11-1998 |
| WO 0000147 | A | 06-01-2000 | DE | 29811372 | U | 05-11-1998 |
| | | | AU | 9745698 | A | 17-01-2000 |
| EP 0948949 | A | 13-10-1999 | US | 6050413 | A | 18-04-2000 |
| US 3638651 | A | 01-02-1972 | DE | 2049493 | A | 15-04-1971 |
| | | | FR | 2065209 | A | 23-07-1971 |
| | | | GB | 1318957 | A | 31-05-1973 |
| | | | SE | 361587 | B | 12-11-1973 |

EPO FORM P0459

For more details about this annex : see Official Journal of the European Patent Office, No. 12/82

17